(19) Europäisches Patentamt
European Patent Office
Office européen des brevets

(11) EP 4 564 354 A1

(12) **EUROPEAN PATENT APPLICATION**

(43) Date of publication:
04.06.2025 Bulletin 2025/23

(21) Application number: 23213426.2

(22) Date of filing: 30.11.2023

(51) International Patent Classification (IPC):
*G16B 30/00* (2019.01)     *C12Q 1/6869* (2018.01)
*G01N 33/487* (2006.01)

(52) Cooperative Patent Classification (CPC):
(C-Sets available)
**G16B 30/00; C12Q 1/6869; G01N 33/48721;
G16B 40/20**                              (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **DNA ME UG**
**22765 Hamburg (DE)**

(72) Inventors:
• **Varlygin, Evgenii**
**01187 Dresden (DE)**
• **Larralde, Mateo**
**01090 Mexico City (MX)**

(74) Representative: **RGTH**
**Patentanwälte PartGmbB**
**Neuer Wall 10**
**20354 Hamburg (DE)**

Remarks:
Amended claims in accordance with Rule 137(2)
EPC.

(54) **METHOD AND SYSTEM FOR BIOPOLYMER SEQUENCING**

(57)    The present invention provides sequencing of biopolymers by: putting a biopolymer in a first chamber of an osmosis cell, the osmosis cell comprising two chambers and a membrane with nanopores; applying a voltage across the osmosis cell; measuring a current through the osmosis cell as the biopolymer passes the nanopores; recording a time sequence of the current; encoding the monomers of the biopolymer via structural electronegativity encoding; and identifying the sequence of the biopolymer from the time sequence of the current using said SEN encoding. Herein, the biopolymer may be any one-dimensional biopolymer, for example, DNA, RNA, proteins, sugars, complex lipids, or artificially created biopolymers.

Fig. 1

EP 4 564 354 A1

**(Cont. next page)**

(52)  Cooperative Patent Classification (CPC): (Cont.)

C-Sets
**C12Q 1/6869, C12Q 2565/607, C12Q 2565/631**

**Description**

This invention relates to the field of biochemistry and in particular to the sequencing of biopolymers including base calling of DNA.

The use of an osmosis cell including a nanopore membrane for the sequencing of nucleotides, or so-called base-calling, is known in the art.

In particular, US 2017 /0370903 A1 discloses multi-cell nanopore-based sequencing chips and methods that can employ formation, characterization, calibration, and/or normalization techniques. For example, various methods may include one or more steps of performing physical checks of cell circuitry, forming and characterizing a lipid layer on the cells, performing a zero point calibration of the cells, forming and characterizing nanopores on the lipid layers of each cell, performing a sequencing operation to accumulate sequencing signals from the cells, normalizing those sequencing signals, and determining bases based on the normalized sequencing signals.

US 2018/0037948 A1 discloses techniques for measuring sequences of nucleic acids. Time-based measurements ( e.g., forming a histogram) particular to a given sequencing cell can be used to generate a tailored model. The model can include probability functions, each corresponding to different states (e.g., different states of a nanopore). Such probability functions can be fit to a histogram of measurements obtained for that cell. The probability functions can be updated over a sequencing run of the nucleic acid so that drifts in physical properties of the sequencing cell can be compensated. A hidden Markov model can use such probability functions for determining the most likely nucleotide states over time. For sequencing cells involving a polymerase, a 2-state classification between bound and unbound states of the polymerase can be performed. The bound regions can be further analyzed by a second classifier to distinguish between states corresponding to different bound nucleotides.

US 2019/0204296 A1 discloses systems and methods for nanopore sequencing base calling. The method can include: receiving raw nanopore sequencing data comprising a plurality of continuous data acquisition (DAC) values corresponding to a biomolecule; normalizing the raw nanopore sequencing data to generate normalized nanopore sequencing data comprising a plurality of normalized DAC values; generating, using a first neural network (NN) and a normalized DAC value of the plurality of normalized DAC values, a vector of transformed probability values, segmenting the plurality of normalized DAC values into a plurality of discrete events; generating, using a second neural network and the event vector, an element determination of the biomolecule.

However, the statistical error and numerical effort of the known methods are in need of improvement.

To improve the sequencing of biopolymers, the present invention provides a method comprising the steps of: putting a biopolymer in a first chamber of an osmosis cell, the osmosis cell comprising two chambers and a membrane with nanopores; applying a voltage across the osmosis cell; measuring a current through the osmosis cell as the biopolymer passes the nanopores; recording a time sequence of the current; encoding the monomers of the biopolymer via structural electronegativity encoding (SEN encoding); and identifying the sequence of the biopolymer from the time sequence of the current using said SEN encoding.

Herein, the biopolymer may be any one-dimensional biopolymer, for example, DNA, RNA, proteins, sugars, complex lipids, or artificially created biopolymers. The biopolymer may be provided in a saline solution in the chamber and the passing of the biopolymer through the nanopores of the membrane may be driven by concentration gradients, by the applied voltage, and/or by use of motor proteins e.g. a DNA polymerase or a topoisomerase attached or tethered to the nanopore.

In another embodiment, the present invention provides a system for determining a sequence of a biopolymer, the system comprising: An osmosis cell comprising two chambers separated by a membrane, wherein one chamber is configured to accept a biopolymer, preferably in saline solution; a voltage source configured to apply a voltage over the osmosis cell; a current measuring device configured to measure a voltage through the osmosis cell; and a control unit configured to: record a time sequence of the current, encode the monomers of the biopolymer via structural electronegativity encoding (SEN encoding); and identify the sequence of the biopolymer from the time sequence of the current using said SEN encoding.

Preferably, the step of identifying the sequence of the biopolymer comprises: In a training phase: Matching the time sequence of the current to a known encoded biopolymer via a neural network; and, in an Identification phase: Using the neural network to translate between a time sequence of a current and the corresponding biopolymer sequence, wherein particularly preferably, the neural network comprises a transformer architecture.

Preferably, the step of matching the time sequence of the current to a known encoded biopolymer comprises a word-piece encoding of the monomers of the biopolymer such that each word-piece token in the neural network corresponds to multiple, preferably three, monomers.

Preferably, the method further comprises using a convolutional layer that can take the average of multiple inputs for word-piece tokens, to adjust to varying compositions of the nucleotides presented inside of the nanopore in the case that the thickness of the nanopore allows for more than one nucleotide per unit of time to pass through the nanopore.

For example, in the case of a nanopore that can contain five nucleotides per each measurement event inside of the pore, the SEN token may be given as some average of the three nucleotide neighbors for each of the subsequent five nucleotides. This average is changed every time one nucleotide passes through the nanopore and another one enters on the other side (i.e. every time the 5-mer sequence inside of the nanopore layer changes).

Furthermore, the method may comprise the step of applying a convolution method to the time sequence of the current and the SEN encoded biopolymer, wherein preferably the convolution method comprises a discrete wavelet transform, DWT.

Preferably, the method comprises using a Neural Network comprising a convolutional layer to process the SEN encoded biopolymer

Preferably, recording the time sequence of the current comprises discretizing the current and/or using a Neural Network, preferably a convoluted or recurrent Neural Network, to synchronize the time sequence of the current with a reference.

Preferably the biopolymer is DNA and the SEN encoding is of length $2^n$, with a natural number n, and particular preferably of length 4 or length 8, numbers per monomer unit, for example per nucleotide base.

Preferably, the SEN encoding of length 4 is given by

| Adenine: | -1; +1; -1; +1 |
| Thymine: | -1; -1; +1; +1 |
| Cytosine: | +1; +1; +1; +1 |
| Guanine: | +1; -1; +1; -1, |

In this case the SEN encoding of length 4 represents the positively and negatively charged domains of the respective nucleotide base as they pass the nanopore.

Also preferably, the SEN encoding of length 8 is given by

| Adenine: | -1; +1; -1; +1; -1; +1; -1; +1, |
| Thymine: | -1; +1; 0; +1; 0; -1; 0; +1, |
| Cytosine: | -1; +1; +1; +1; +1; -1; 0; +1, and |
| Guanine: | 0; +1; -1; +1; -1; -1; +1; -1, |

In this case the SEN encoding corresponds to a finer-grained mapping of the neutral and positively and negatively charged domains of the corresponding nucleotide bases.

Preferably, the method further comprises using an additional neural network layer to adjust the SEN encoding to increase the accuracy after a first approximation, e.g. taking the encodings of length 4 or 8 given above as a starting approximation, wherein said additional neural network layer is trained in coherence with the neural network trained in the training phase.

Still further preferably a user interface to find patterns and for cluster analysis may be provided.

Preferably the control unit of the system according to an embodiment of the invention comprises a neural network configured to: in a training phase: Matching the time sequence of the current to a known encoded biopolymer via a neural network; and in an Identification phase: Use the neural network to translate between a time sequence of a current and the corresponding biopolymer sequence.

Further preferably, the control unit is configured to apply a word- piece encoding of the monomers of the biopolymer such that each word-piece token in the neural network corresponds to multiple, preferably three monomers, and the control unit is preferably further configured to apply a convolution method, to the time sequence of the current and the SEN encoded biopolymer, wherein preferably the convolution method comprises a discrete wavelet transform, DWT.

Preferably, the control unit further performs discretizing of the time sequence of the current and/or comprises a convoluted Neural Network configured to perform synchronizing the time sequence of the current with a reference.

Preferably, wherein the biopolymer is DNA and the SEN encoding is preferably given by:

| Adenine: | -1; +1; -1; +1; -1; +1; -1; +1, |
| Thymine: | -1; +1; 0; +1; 0; -1; 0; +1, |
| Cytosine: | -1; +1; +1; +1; +1; -1; 0; +1, and |
| Guanine: | 0; +1; -1; +1; -1; -1; +1; -1. |

Preferably, the control unit is further configured to train an additional neural network layer to adjust the SEN encoding to increase accuracy after the first approximation, wherein said additional neural network layer is trained in coherence with the neural network trained in the training phase.

Due to inconsistent dynamics such as slippage, sticking, or quasi-static behavior in homogeneous regions (which are particularly projected in the time domain as stochastic artefacts), statistical methods that can resolve such artefacts via normalization procedures or "event-context" adaptations employing one-dimensional convolutional neural networks, Transformer Neural Networks (in the following abbreviated as NN), or Deep associative NN, may be applied. The dynamic-adapted current sequence may then be synchronized and calibrated with the reference electrostatic pattern encoding of the biopolymer by wavelet decomposition.

The encoding method, named as Structural Electronegativity (SEN), encodes after a vital marker of the metabolism (living activity) and/or catabolism (degeneration), and is particularly involved in the mechanism of recognition of biopolymer structural units during transcription, translation, and post-translational modifications.

Structural electronegativity is the tendency of a molecular structure to be recognized as an informational interface of cellular compartments via electrostatic forces. It is a measure of the atom's ability to push or pull electrons towards or away from other atoms in the molecule. Within the context of DNA and/or RNA sequencing, purines and pyrimidines are nitrogenous bases responsible for carrying genetic information and catalyzing chemical reactions.

Besides the explicit mechanism of storing information in pair-wise matching of the nitrogenous bases for translation/transcription of cell machineries (Watson-Crick model), which are presented as static properties, SEN patterns could also participate in dynamic interaction with the corresponding biopolymer. Particularly, it is expected that biopolymer units reflect cis/trans heterogeneity of unit conformation with respect to the normal biopolymer's polarity.

The SEN method provides a mechanism of recognition of Nucleic Acids, and my also be used as a method to recognize epigenetic markers, controlled via methylation/demethylation modifications. These modifications could be defined as a 'dynamic lockage' of cis/trans conformations in quasi-stable segments (i.e., low number of molecular events is that segment), leading to control of "visible" or "readable" fragments in recognition processes. Such "lockage" can be described analogously to a "key - lock" mechanism: the corresponding electronegativity field of the matching biopolymer units fits into the specific segment. Additionally, methylation within the segment result in changes of the electronegativity field, which in turn affects the recognition side which can lead to signaling of suppression or expression of the gene.

For methylated fragments, this can prevent motor protein rotation that holds a candidate to match to move along with matching elements from the recognition side of the biopolymer (key is not matching to lock teeth pattern that leads to stuck of rotation inside of the lock cell).

For unmethylated fragments, not constrained via mechanisms described above, in case of matching a candidate the whole structure, consisting of the candidate, recognition side units, and motor protein rotating elements are believed to move synchronously (key turns with teeth of the lock, providing atomic force feedback to cell agent that the match was correct).

The invention will be further explained in more detail with reference to the figures.

Fig. 1 shows a "word-piece" encoding of a string of DNA.
Fig. 2 shows the SEN encoding of individual nucleotide bases.
Fig. 3 shows the use of SEN encoding and DWT decomposition to obtain SEN positional encoding.
Figs. 4a to 4c show the steps of feature extraction, dictionary derivation and context tuning in accordance with an embodiment.
Fig. 5 shows an overview of a sequence analyzer in accordance with an embodiment.

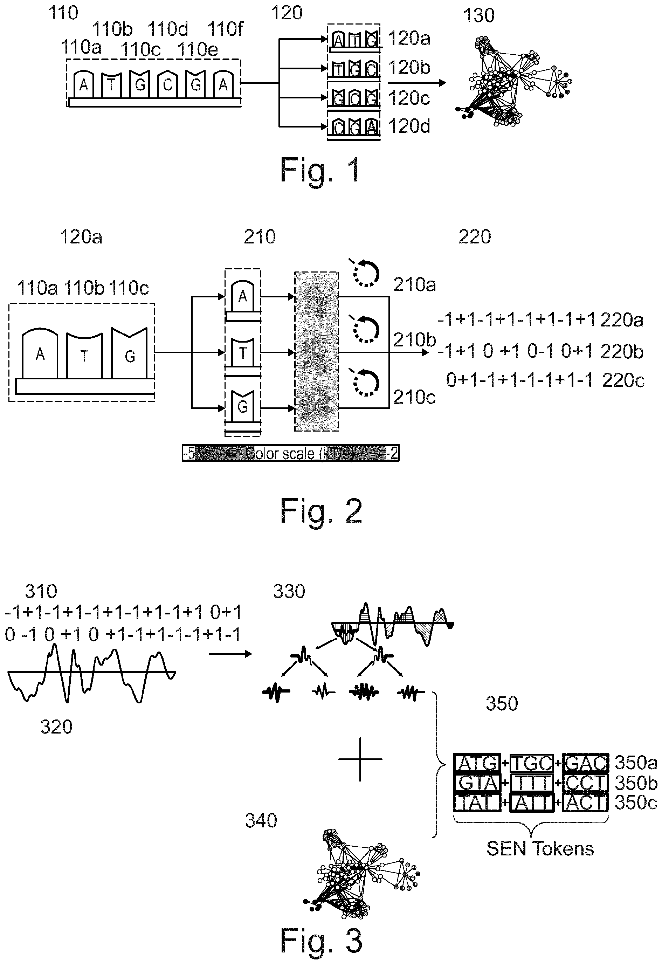

As shown in Fig. 1, when encoding a biopolymer, e.g. a string 110 of nucleotides 110a-110f using the SEN scheme, the most basic information unit ("word-piece") is of a nucleotide triplet 120 (as it was shown in literature that only the closest 2 neighbors affect electrostatic properties of 1d biopolymers). This gives a total of $4^3 = 64$ possible "word-piece" combinatoric variations (AAA, AAT, AAG, ... etc).

Each "word-piece" token 120a-120d is labeled by an integer number and represented by a multi vector trainable embedding of context clusters 130, the embedding projecting relevant features and properties that later are converged with electrical signal signatures.

As shown in Fig. 2, to obtain a physically objective encoding of the investigated biopolymer 110a-c in synchronization with its physical properties, a series of electrostatic simulations 210a-c are conducted using the Poisson-Boltzmann electrostatic potential model with respect to the biopolymer informational polarity (3'-5' end of Nucleic Acid direction, C-N terminuses of Amino acid etc.). Particularly, if the biopolymer structural unit has fluctuations of the electrostatic potential, said fluctuations may interact with a point positive charge (repulsion, or negative-outward force drift in case of positively charged atom of the biopolymer; attraction, or positive-toward force drift in case of negatively charged atom of the biopolymer; and a neutral area in between positive and negative charges of the molecule) and thus may produce signals in the measured current when the biopolymer passes through the nanopore (positive current spike for negatively charged atom, negative current spike for positively charged atom). This encoding scheme 220 results in the splitting of electrostatic potential domains into discrete numbers 220a-c representing respective direction of point charge drift (+1 for negative electrostatic field domain, -1 for positive one and 0 for neutral one).

Such encoding methodology allows the representation of the 4 DNA nucleobases as the following matrix:

$$( 0, +1, -1, +1, -1, -1, +1, -1) = G$$

$$(-1, +1, -1, +1, -1, +1, -1, +1) = A$$

$$(-1, +1, 0, +1, 0, -1, 0, +1) = T$$

$$(-1, +1, +1, +1, +1, -1, 0, +1) = C$$

The encoding according to an embodiment of the invention provides a possibility to represent biopolymer modification, such as methylation, and it also allows the identification of an "essential factor" property of the biopolymers, that can interrelate and easily contrast biopolymer units with each other, and extract their electrostatic similarity.

The described encoding method contributes significantly as an extra mathematical term in the calculation of the "context landscape" of a Transformer NN (or any other big scale NN model) architecture, used as an engine for the biomarker search method of the present invention and for raw electrical signal filter generative methodology.

As shown in Fig. 3, after the representation of each structural unit via SEN encoding 310, a Discrete Wavelet Transformations 330 on 16 (may vary) frequency sub bands with kernel size of 16 units and shift of sliding window of 4 (half of the 8 SEN encoding numbers; such shift mechanism allows to store transition changes from nucleotide to nucleotide stored as discrete values of DWT decomposition) may be applied to the SEN encoded structural units. The result may then be finalized by a parallelized Convolutional layer 340 of a NN with an amount of groups equal to the number of sub band channels, and a kernel size able to capture at least a receptive field equivalent to the representation of 3 sequential nucleotides, resulting in "Structural Electronegativity Positional Encoding" 350.

Together, the "word-piece" 120 embedding tokens and the above-described SEN, respectively the above-described "Structural Electronegativity Positional Encoding" 350 may be used in a linear summation with respect to the position in the biopolymer 110; this represents a unified input for encoding within the neural network machinery. The decoding input is comprised of a direct DWT decomposition of the raw current signal 320. As it is natively represented by physical parameters, and because of the nanopore sequencing measurement methodology (recording of the flow of ionic current, which serves as a projection of the electrostatic potential surfaces of positive point charges attracted by different parts of the biopolymer), such input already consists of the two components described above for encoding input, and only needs corrections for synchronization with the encoding reference sub-band channels and the vectorized patterns of the "word-piece" tokens 120.

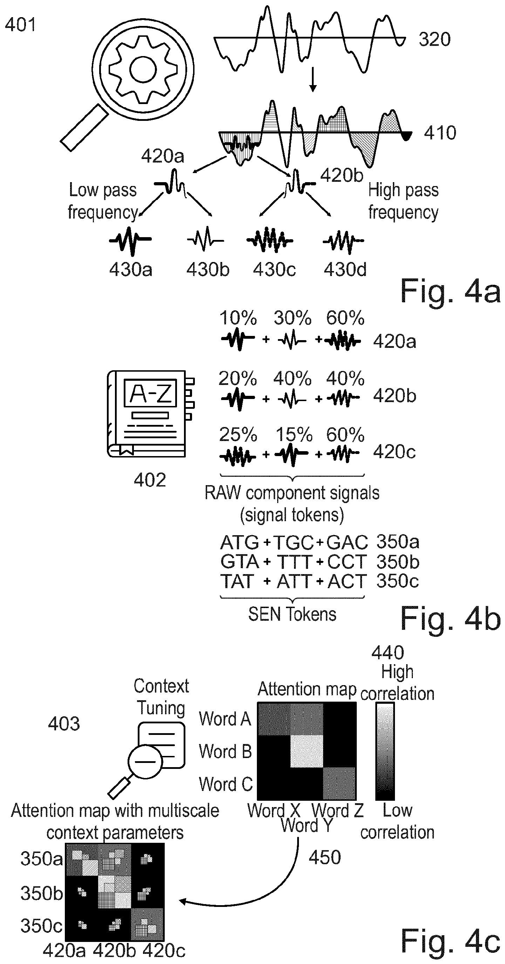

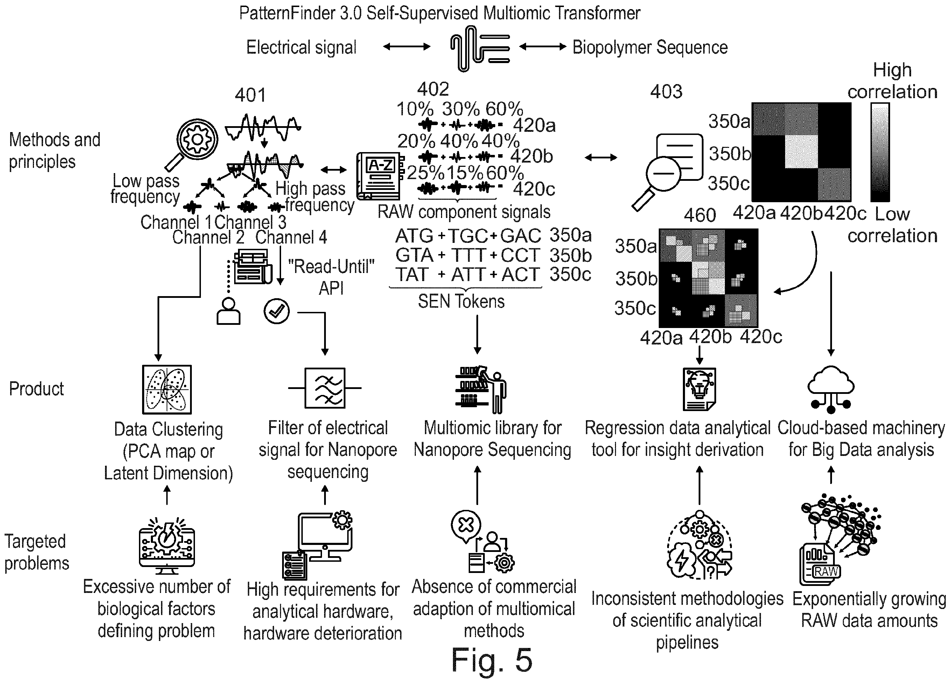

Figs. 4a to 4c and 5 describe the methodology of an embodiment of the present invention in terms of the following sequential steps:

Step 1: Feature Extraction 401

As illustrated by Figure 4a, the 1D signal 320 that is obtained from the reading of the biopolymer current signal is convolved via 1D convolution 410 with a characteristic wavelet (Discrete Wavelet Transformation) into the batch of component signals 420a-b representing multi-frequency sampling of the original raw signal. These component signals are separated into different channels 430a-d according to their frequency.

Step 2: Dictionary Derivation 402

As shown in Fig. 4b, the component signals 420 a-c obtained from the raw signal are then synchronized with the dictionary units that will ensemble into the signature signals.

In addition, another dictionary 350a-c consisting of combinatorial sequences (structured by SEN tokens) of the biopolymer's intrinsic units 110 (e.g., nucleotide abbreviations for DNA or RNA nucleotides, amino acids for proteins, etc. including their quantized electrostatic point potentials) is also derived. The regressions between the signal token dictionary and the biopolymer SEN token dictionary are derived via the process of Context Tuning and later used for the process of translation.

Step 3: Context Tuning 403

As shown in Fig. 4c, to cross-correlate the two different types of dictionaries 350, 420 , a calibration method to obtain an optimal translation mechanism is required. This method provides a ranking system 440 of the tokens (filtering of irrelevant tokens from final dictionaries), defining their statistical contribution to decision making in the translation process. During the training process, the proposed invention uses a processing method 450 based on Transformer Machine Learning (TML) architecture and statistical re-parametrization of sampled dictionary tokens.

As shown in Fig. 5, the TML provides a graphical representation of the regression mechanism (Attention map) of dictionaries 350, 420 and their units. This can be used as a calibration mechanism for fine-tuning of the Machine Learning architecture parameters and later serves as a front-end mechanism for "Data Clustering" and "PatternFinder" parts of a graphical user interface .

List of References:

| | |
|---|---|
| 110 | string of nucleotides |
| 110a-f | nucleotides |
| 120 | "word-piece" nucleotide triplet |
| 120a-d | word-pieces |
| 130 | context cluster |
| 210 | electrostatic simulation |
| 210a-c | electrostatic simulations of nucleotides |
| 220 | SEN encoding |
| 220a-c | discrete numbers representing charge domains |
| 310 | SEN encoding |
| 320 | current signal |
| 330 | Discrete Wavelet Transformation |
| 340 | convolutional layer |
| 350 | Structural Electronegativity Positional Encoding |
| 350a-c | directory of combinatorial sequences |
| 401 | feature extraction |
| 402 | dictionary derivation |
| 403 | context tuning |
| 410 | Convolution of current and wavelet |
| 420a-c | component signals |
| 430a-c | frequency channels |
| 440 | ranking of tokens |
| 450 | transformer |

**Claims**

1.  A method for determining a sequence of a biopolymer, the method comprising:

    putting a biopolymer in a first chamber of an osmosis cell, the osmosis cell comprising two chambers and a membrane with nanopores;
    applying a voltage across the osmosis cell;
    measuring a current through the osmosis cell as the biopolymer passes the nanopores from the first chamber to a second chamber;
    recording a time sequence of the current;
    encoding the monomers of the biopolymer via structural electronegativity encoding (SEN encoding); and
    identifying the sequence of the biopolymer from the time sequence of the current using said SEN encoding.

2.  The method of claim 1, wherein the step of identifying the sequence of the biopolymer comprises

    in a training phase: Matching the time sequence of the current to a known encoded biopolymer via a neural network; and
    in an Identification phase: Using the neural network to translate between a time sequence of a current and the corresponding biopolymer sequence,

wherein the neural network preferably comprises a transformer architecture.

3. The method of claim 2, wherein the step of matching the time sequence of the current to a known encoded biopolymer comprises a word-piece encoding of the monomers of the biopolymer such that each word-piece token in the neural network corresponds to multiple, preferably three, monomers.

4. The method of claim 3, further comprising applying a convolution method to the time sequence of the current and the SEN encoded biopolymer, wherein the convolution method is preferably a discrete wavelet transform, DWT.

5. The method of any of claims 2 to 4, further comprising using a Neural Network comprising a convolutional layer to process the SEN encoded biopolymer.

6. The method of any of the preceding claims, wherein recording the time sequence of the current comprises discretizing the current and/or using a Neural Network, preferably a convoluted or recurrent Neural Network to synchronize the time sequence of the current with a reference.

7. The method of any of the preceding claims, wherein the biopolymer is DNA or RNA, preferably including respective methylation and post-modification profiles.

8. The method of claim 7, wherein the SEN encoding is of length $2^n$ with a natural number n, preferably of length 4 or length 8, numbers per monomer unit.

9. The method of claim 8, wherein the length of the SEN encoding is 8 numbers per monomer unit and the SEN encoding is given by

| | |
|---|---|
| Adenine: | -1; +1; -1; +1; -1; +1; -1; +1, |
| Thymine: | -1; +1; 0; +1; 0; -1; 0; +1, |
| Cytosine: | -1; +1; +1; +1; +1; -1; 0; +1, and |
| Guanine: | 0; +1; -1; +1; -1; -1; +1; -1, and/or |

wherein the method further comprises using an additional neural network layer to adjust the SEN encoding to increase the accuracy after a first approximation, wherein said additional neural network layer is trained in coherence with the neural network trained in the training phase.

10. The method of any of the preceding claim, further providing a user interface to find patterns and for cluster analysis.

11. A system for determining a sequence of a biopolymer, the system comprising:

An osmosis cell comprising two chambers separated by a membrane, wherein one chamber is configured to accept a biopolymer, preferably in saline solution;
A voltage source configured to apply a voltage over the osmosis cell;
A current measuring device configured to measure a voltage through the osmosis cell; and
a control unit configured to:

record a time sequence of the current,
encode the monomers of the biopolymer via structural electronegativity encoding (SEN encoding); and
identify the sequence of the biopolymer from the time sequence of the current using said SEN encoding.

12. The system of claim 11, wherein the control unit comprises a neural network configured to:

in a training phase: Matching the time sequence of the current to a known encoded biopolymer via a neural network; and
in an Identification phase: Use the neural network to translate between a time sequence of a current and the corresponding biopolymer sequence.

13. The system of claim 12, wherein the control unit is further configured to apply a word-piece encoding of the monomers of the biopolymer such that each word-piece token in the neural network corresponds to multiple, preferably three monomers and wherein the control unit is preferably further configured to apply a discrete wavelet transform, DWT, to the time sequence of the current and the SEN encoded biopolymer.

14. The system of one of claims 11 to 13, wherein the control unit further performs discretizing of the time sequence of the current and/or comprises a convoluted Neural Network configured to perform synchronizing of the time sequence of the current with a reference.

15. The system of one of claims 11 to 13, wherein the biopolymer is DNA and the SEN encoding is preferably given by

| | |
|---|---|
| Adenine: | -1; +1; -1; +1; -1; +1; -1; +1, |
| Thymine: | -1; +1; 0; +1; 0; -1; 0; +1, |
| Cytosine: | -1; +1; +1; +1; +1; -1; 0; +1, and |
| Guanine: | 0; +1; -1; +1; -1; -1; +1; -1 and/or |

wherein the control unit is further configured to use an additional neural network layer to adjust the SEN encoding to increase the accuracy after a first approximation, wherein said additional neural network layer is trained in coherence with the neural network trained in the training phase.

**Amended claims in accordance with Rule 137(2) EPC.**

1. A method for determining a sequence of a biopolymer, the method comprising:

   putting a biopolymer in a first chamber of an osmosis cell, the osmosis cell comprising two chambers and a membrane with nanopores;
   applying a voltage across the osmosis cell;
   measuring a current through the osmosis cell as the biopolymer passes the nanopores from the first chamber to a second chamber;
   recording a time sequence of the current;
   encoding the monomers of the biopolymer via structural electronegativity encoding (SEN encoding), wherein the SEN encoding represents the positively and negatively charged domains, and preferably the neutral domains, of the respective nucleotide base which produce signals in the measured current as they pass the nanopore; and
   identifying the sequence of the biopolymer from the time sequence of the current using said SEN encoding,
   wherein the step of identifying the sequence of the biopolymer comprises in a training phase: Matching the time sequence of the current to a known encoded biopolymer via a neural network; and
   in an Identification phase: Using the neural network to translate between a time sequence of a current and the corresponding biopolymer sequence,
   wherein the neural network preferably comprises a transformer architecture.

2. The method of claim 1, wherein the step of matching the time sequence of the current to a known encoded biopolymer comprises a word-piece encoding of the monomers of the biopolymer such that each word-piece token in the neural network corresponds to multiple, preferably three, monomers.

3. The method of claim 2, further comprising applying a convolution method to the time sequence of the current and the SEN encoded biopolymer, wherein the convolution method is preferably a discrete wavelet transform, DWT.

4. The method of any of claims 1 to 3, further comprising using a Neural Network comprising a convolutional layer to process the SEN encoded biopolymer.

5. The method of any of the preceding claims, wherein recording the time sequence of the current comprises discretizing the current and/or using a Neural Network, preferably a convoluted or recurrent Neural Network to synchronize the time sequence of the current with a reference.

6. The method of any of the preceding claims, wherein the biopolymer is DNA or RNA, preferably including respective methylation and post-modification profiles.

7. The method of claim 6, wherein the SEN encoding is of length $2^n$ with a natural number n, preferably of length 4 or length 8, numbers per monomer unit.

8. The method of claim 7, wherein the length of the SEN encoding is 8 numbers per monomer unit and the SEN encoding is given by

Adenine: -1; +1; -1; +1; -1; +1; -1; +1,
Thymine: -1; +1; 0; +1; 0; -1; 0; +1,
Cytosine: -1; +1; +1; +1; +1; -1; 0; +1, and
Guanine: 0; +1; -1; +1; -1; -1; +1; -1, and/or
wherein the method further comprises using an additional neural network layer to adjust the SEN encoding to increase the accuracy after a first approximation, wherein said additional neural network layer is trained in coherence with the neural network trained in the training phase.

9. The method of any of the preceding claim, further providing a user interface to find patterns and for cluster analysis.

10. A system for determining a sequence of a biopolymer, the system comprising:

An osmosis cell comprising two chambers separated by a membrane, wherein one chamber is configured to accept a biopolymer, preferably in saline solution;
A voltage source configured to apply a voltage over the osmosis cell;
A current measuring device configured to measure a voltage through the osmosis cell; and
a control unit configured to:

record a time sequence of the current,
encode the monomers of the biopolymer via structural electronegativity encoding (SEN encoding); and
identify the sequence of the biopolymer from the time sequence of the current using said SEN encoding, wherein the SEN encoding represents the positively and
negatively charged domains, and preferably the neutral domains, of the respective nucleotide base produce signals in the measured current as they pass the nanopore, and
wherein the control unit further comprises a neural network configured to:

in a training phase: Matching the time sequence of the current to a known encoded biopolymer via a neural network; and
in an Identification phase: Use the neural network to translate between a time sequence of a current and the corresponding biopolymer sequence.

11. The system of claim 10, wherein the control unit is further configured to apply a word-piece encoding of the monomers of the biopolymer such that each word-piece token in the neural network corresponds to multiple, preferably three monomers and wherein the control unit is preferably further configured to apply a discrete wavelet transform, DWT, to the time sequence of the current and the SEN encoded biopolymer.

12. The system of claim 10 or 11, wherein the control unit further performs discretizing of the time sequence of the current and/or comprises a convoluted Neural Network configured to perform synchronizing of the time sequence of the current with a reference.

13. The system of one of claims 10 to 12, wherein the biopolymer is DNA and the SEN encoding is preferably given by

Adenine: -1; +1; -1; +1; -1; +1; -1; +1,
Thymine: -1; +1; 0; +1; 0; -1; 0; +1,
Cytosine: -1; +1; +1; +1; +1; -1; 0; +1, and
Guanine: 0; +1; -1; +1; -1; -1; +1; -1 and/or
wherein the control unit is further configured to use an additional neural network layer to adjust the SEN encoding to increase the accuracy after a first approximation, wherein said additional neural network layer is trained in coherence with the neural network trained in the training phase.

110

110b 110d 110f
110a 110c 110e

120

120a
120b
120c
120d

130

## Fig. 1

120a

110a 110b 110c

A T G

210

A

T

G

210a

210b

210c

220

-1+1-1+1-1+1-1+1 220a

-1+1 0 +1 0-1 0+1 220b

0+1-1+1-1-1+1-1 220c

-5   Color scale (kT/e)   -2

## Fig. 2

310
-1+1-1+1-1+1-1+1-1+1 0+1
0 -1 0 +1 0 +1-1+1-1-1+1-1

320

330

350

340

+

ATG+TGC+GAC 350a
GTA+TTT+CCT 350b
TAT+ATT+ACT 350c

SEN Tokens

## Fig. 3

401

320

410

420a

Low pass
frequency

420b

High pass
frequency

430a    430b    430c    430d

# Fig. 4a

10%   30%   60%
+    +    420a

20%   40%   40%
+    +    420b

25%   15%   60%
+    +    420c

402

RAW component signals
(signal tokens)

ATG + TGC + GAC  350a
GTA + TTT + CCT  350b
TAT + ATT + ACT  350c

SEN Tokens

# Fig. 4b

440
High
correlation

Context
Tuning

403

Attention map

Word A
Word B
Word C

Word X  Word Z
Word Y

Low
correlation

Attention map with multiscale
context parameters

450

350a
350b
350c

420a 420b 420c

# Fig. 4c

PatternFinder 3.0 Self-Supervised Multiomic Transformer

Electrical signal ◄──► ◄──► Biopolymer Sequence

**401**

Methods and principles

Low pass frequency   High pass frequency
Channel 1 Channel 3
Channel 2   Channel 4

"Read-Until" API

**402**
10% 30% 60%
──── 420a
20% 40% 40%
──── 420b
25% 15% 60%
──── 420c
RAW component signals

ATG + TGC + GAC  350a
GTA + TTT + CCT  350b
TAT + ATT + ACT  350c
SEN Tokens

**403**

350a
350b
350c

High correlation

460   420a 420b 420c   Low correlation

350a
350b
350c
420a 420b 420c

Product

Data Clustering (PCA map or Latent Dimension)

Filter of electrical signal for Nanopore sequencing

Multiomic library for Nanopore Sequencing

Regression data analytical tool for insight derivation

Cloud-based machinery for Big Data analysis

Targeted problems

Excessive number of biological factors defining problem

High requirements for analytical hardware, hardware deterioration

Absence of commercial adaption of multiomical methods

Inconsistent methodologies of scientific analytical pipelines

Exponentially growing RAW data amounts

Fig. 5

Europäisches
Patentamt

European
Patent Office

Office européen
des brevets

## EUROPEAN SEARCH REPORT

**Application Number**

EP 23 21 3426

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/213541 A1 (BROWN CLIVE GAVIN [GB] ET AL) 7 July 2022 (2022-07-07) * paragraphs [0191] - [0510] * | 1-15 | INV.<br>G16B30/00<br>C12Q1/6869<br>G01N33/487 |
| A | US 2022/366313 A1 (GUNDLACH JENS H [US] ET AL) 17 November 2022 (2022-11-17) * paragraphs [0075] - [0395] * | 1-15 | |
| A | WO 2023/183937 A1 (ILLUMINA INC [US]; ILLUMINA SOFTWARE INC [US]) 28 September 2023 (2023-09-28) * paragraphs [0095] - [0268] * | 1-15 | |
| A | US 2020/333290 A1 (LEBURTON JEAN-PIERRE [US] ET AL) 22 October 2020 (2020-10-22) * paragraphs [0020] - [0094]; figure 10 * | 1-15 | |
| A | Anonymous: "Nanopore sequencing", , 17 September 2023 (2023-09-17), XP093162879, Retrieved from the Internet: URL:https://en.wikipedia.org/w/index.php?title=Nanopore_sequencing&oldid=1175741132 * the whole document * | 1-15 | **TECHNICAL FIELDS SEARCHED (IPC)**<br><br>G16B<br>G01N<br>C12Q |
| A | NAKANE J J ET AL: "Nanopore sensors for nucleic acid analysis", JOURNAL OF PHYSICS: CONDENSED MATTER, INSTITUTE OF PHYSICS PUBLISHING, BRISTOL, GB, vol. 15, 1 August 2003 (2003-08-01), pages R1365-R1393, XP007902737, ISSN: 0953-8984, DOI: 10.1088/0953-8984/15/32/203 * the whole document * | 1-15 | |

The present search report has been drawn up for all claims

| Place of search | Date of completion of the search | Examiner |
|---|---|---|
| Munich | 15 May 2024 | Díaz de Lezana, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 23 21 3426

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2024

| Patent document cited in search report | Publication date | Patent family member(s) | | Publication date |
|---|---|---|---|---|
| US 2022213541 A1 | 07-07-2022 | CN | 112703256 A | 23-04-2021 |
| | | EP | 3847278 A1 | 14-07-2021 |
| | | JP | 7408665 B2 | 05-01-2024 |
| | | JP | 2021534831 A | 16-12-2021 |
| | | KR | 20210055690 A | 17-05-2021 |
| | | US | 2022213541 A1 | 07-07-2022 |
| | | WO | 2020049293 A1 | 12-03-2020 |
| US 2022366313 A1 | 17-11-2022 | US | 2022366313 A1 | 17-11-2022 |
| | | WO | 2020168286 A1 | 20-08-2020 |
| WO 2023183937 A1 | 28-09-2023 | US | 2023343414 A1 | 26-10-2023 |
| | | WO | 2023183937 A1 | 28-09-2023 |
| US 2020333290 A1 | 22-10-2020 | NONE | | |

EPO FORM P0459

For more details about this annex : see Official Journal of the European Patent Office, No. 12/82

## REFERENCES CITED IN THE DESCRIPTION

**Patent documents cited in the description**

- US 20170370903 A1
- US 20180037948 A1
- US 20190204296 A1